(19) Europäisches Patentamt
European Patent Office
Office européen des brevets

(11) Numéro de publication : **0 441 690 A2**

(12) **DEMANDE DE BREVET EUROPEEN**

(21) Numéro de dépôt : **91400256.3**

(22) Date de dépôt : **04.02.91**

(51) Int. Cl.$^5$ : **C08B 37/16,** // A61K9:18, A23L1:277, C08F4:34, A61K7:50

(30) Priorité : **09.02.90 FR 9001506**

(43) Date de publication de la demande :
**14.08.91 Bulletin 91/33**

(84) Etats contractants désignés :
**AT BE CH DE DK ES FR GB GR IT LI LU NL SE**

(71) Demandeur : **RHONE-POULENC CHIMIE**
**25, quai Paul Doumer**
**F-92408 Courbevoie Cédex (FR)**

(72) Inventeur : **Dalbe, Bernard**
**53 Boulevard Marcel Sembat**
**F-93200 Saint Denis Cédex (FR)**
Inventeur : **Wittig, Etienne**
**13, rue Serge**
**F-77500 Chelles (FR)**

(74) Mandataire : **Seugnet, Jean Louis et al**
**RHONE-POULENC CHIMIE SERVICE**
**BREVETS CHIMIE 25 Quai Paul Doumer**
**F-92408 Courbevoie Cédex (FR)**

(54) **Composés d'inclusion de cyclodextrines enfermant des peroxydes organiques, procédés de préparation et utilisations.**

(57) L'invention concerne des composés d'inclusion de cyclodextrines enfermant des peroxydes organiques de formule $R_1$-O-O-$R_2$ dans laquelle $R_1$ et $R_2$ sont identiques ou différents et représentent

$$R_3 \text{ ou } R_3-\underset{\underset{O}{\|}}{C},$$

$R_3$ étant un reste hydrocarbonné. L'invention concerne également des procédés de préparation desdits composés d'inclusion, ainsi que leurs utilisations dans l'industrie pharmaceutique, cosmétique ou alimentaire et en tant qu'initiateur de polymérisation ou de copolymérisation.

**EP 0 441 690 A2**

Jouve, 18, rue Saint-Denis, 75001 PARIS

# COMPOSES D'INCLUSION DE CYCLODEXTRINES ENFERMANT DES PEROXYDES ORGANIQUES, PROCEDES DE PREPARATION ET UTILISATIONS

La présente invention concerne des composés d'inclusion de cyclodextrines enfermant des peroxydes organiques, des procédés de préparation desdits composés d'inclusion ainsi que leurs utilisations dans l'industrie pharmaceutique, cosmétique ou alimentaire, et en tant qu'initiateur de polymérisation ou de copolymérisation.

Les cyclodextrines sont des oligosaccharides cycliques composés de résidus glucoses liés par des liaisons $\alpha \rightarrow 1,4$ et comportant une cavité. On distingue généralement trois types de cyclodextrines : les cyclodextrines $\alpha$, constituées de six résidus glucoses, les cyclodextrines $\beta$, constituées de sept résidus glucoses et les cyclodextrines $\gamma$ constituées de huit résidus glucoses. Des cyclodextrines $\delta$, comportant 9 résidus glucoses ont également été décrites. Les cyclodextrines peuvent être préparées à partir d'amidons d'origines diverses, modifiés ou non modifiés, de glycogène, ou de fractions d'amidons tels les maltodextrines, les amyloses ou les amylopectines. Ces produits de départ sont liquifiés par exemple par gélatinisation ou par traitement au moyen d'une enzyme de liquéfaction telle une $\alpha$-amylase, puis sont soumis à l'action d'une cyclodextrine-glucosyl-transférase (CGTase). On peut citer plus particulièrement le procédé de préparation de cyclodextrines décrit dans US 3.923.598 selon lequel la CGTase employée est produite par des souches Bacillus sp. spécifiques. Ces procédés permettent généralement l'obtention d'un mélange de cyclodextrines $\alpha$, $\beta$ et $\gamma$ en des proportions variables. Il est cependant possible d'isoler chaque type de cyclodextrine selon des méthodes classiques ou par exemple selon les procédés décrits dans les brevets US 4.418.144 et US 4.384.898.

On connaît d'autres cyclodextrines, qui sont des cyclodextrines modifiées, généralement obtenues à partir desdites cyclodextrines $\alpha$, $\beta$ et $\gamma$. Parmi ces cyclodextrines on peut citer celles modifiées par des groupes acyles, alkyles, hydroxyalkyles, glucosyles ou maltosyles. Ces cyclodextrines modifiées peuvent être obtenues par les procédés notamment décrits dans la demande de brevet français N° 2 597 785, dans "cyclodextrins and their industrial uses" Dominique Duchêne, ed. de Santé, 1987, pages 366-388 ou dans "Cyclodextrin Technology", Jozsef Szejtli, ed. Kluwer Academic Publishers, page 2.

Les cyclodextrines sont généralement utilisées soit pour former des poudres de parfums, de thé, d'épices, d'insecticides, de fongicides, d'arômes ou de vitamines liposolubles, et ainsi préserver les propriétés de ces produits, soit pour éliminer des composants amers présents par exemple dans les agrumes.

Les peroxydes organiques sont des composés de formule générale

$$R_1\text{-O-O-}R_2$$

dans laquelle $R_1$ et $R_2$ sont identiques ou différents et représentent un reste $R_3$ ou un reste

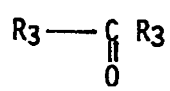

étant un reste hydrocarboné.

Les peroxydes organiques peuvent se décomposer facilement par un apport d'énergie sous forme d'une source de chaleur, de lumière ou d'un choc mécanique comme un impact ou un friction. Il est donc recommandé de stocker et de manipuler lesdits peroxydes en prenant beaucoup de précautions. De plus, la plupart des peroxydes organiques sont toxiques et irritants pour la peau et les yeux, rendant leur utilisation délicate. Enfin, les peroxydes organiques sont peu solubles dans l'eau. Une meilleure solubilité desdits peroxydes organiques peut modifier leur utilisation dans certaines applications notamment dans leur emploi en tant qu'initiateur de polymèrisation ou de copolymérisation.

Un premier objet de la présente invention consiste en des composés comportant de tels peroxydes organiques de sorte à les rendre moins sensibles à la décomposition, qui permettent de réduire leur toxicité et leur effet irritant et qui les rendent plus solubles en milieu aqueux.

Un deuxième objet de l'invention consiste en des procédés de préparation de tels composés.

Un dernier objet de l'invention concerne l'utilisation desdits composés dans l'industrie pharmaceutique, cosmétique et alimentaire et en tant qu'initiateur de polymérisation ou de copolymérisation.

La présente invention concerne donc des composés d'inclusion de cyclodextrines caractérisés en ce qu'ils consistent en des molécules de cyclodextrines enfermant des peroxydes organiques de formules $R_1\text{-O-O-}R_2$ dans laquelle $R_1$ et $R_2$ sont identiques ou différents et représentent un reste $R_3$ ou

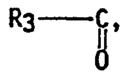

$R_3$ étant un reste hydrocarboné.

Par le terme "enfermant" on entend dans le cadre de la présente invention, qu'au moins une partie de la molécule dudit peroxyde organique est inclue dans la cavité de la molécule de cyclodextrine.

Lesdits composés d'inclusion de cyclodextrines peuvent être constitués à partir de cyclodextrines α, β, γ ou δ ou de cyclodextrines α, β, γ ou δ modifiées par des groupes acyles, alkyles, hydroxyalkyles, glucosyles ou maltosyles. On peut également utiliser des mélanges de telles cyclodextrines ou des mélanges de cyclodextrines et de dextrines ou d'oligosaccharides linéaires. On préfère toutefois les cyclodextrines β.

Lesdits peroxydes organiques sont de préférence des molécules dans lesquelles $R_3$ est un radical aliphatique linéaire ou ramifié, saturé ou insaturé ou un radical aryle.

Avantageusement $R_3$ peut être un radical alkyle en $C_1$-$C_{10}$ ou un radical phényle. Les composés d'inclusion selon la présente invention conviennent plus particulièrement pour les peroxydes organiques dans lesquelles $R_1$ et $R_2$ représentent un reste

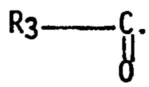

De tels peroxydes organiques peuvent être en particulier le peroxyde de dibenzoyle ou le peroxyde de dioctanoyle.

Lesdits composés d'inclusion de cyclodextrines peuvent comporter plus de 5 %, généralement de 10 à 30 % en poids de peroxydes organiques par rapport au poids total du composé d'inclusion.

Les composés d'inclusion selon l'invention se présentent généralement sous forme solide et pulvérulente.

L'invention concerne également des procédés de préparation desdits composés d'inclusion. Selon un premier procédé de préparation on effectue les étapes suivantes :

a) On solubilise les cyclodextrines dans un solvant dans lequel on ajoute le peroxyde organique antérieurement, simultanément ou postérieurement auxdites cyclodextrines,

b) On porte le mélange ainsi obtenu à une température comprise entre 25 et 70° C, de préférence comprise entre 40 et 65° C,

c) On récupère le composé d'inclusion ainsi formé,

Le solvant utilisé pour solubiliser les cyclodextrines peut être de l'eau ou un mélange d'eau et d'au moins un solvant organique miscible dans l'eau. De tels solvants organiques peuvent être des alcools tels le méthanol, l'éthanol, le n-propanol ou l'isopropanol, des cétones telle l'acétone ou des éthers tel le dioxanne. Lesdits alcools sont toutefois préférés. Lorsqu'on utilise un tel mélange le rapport pondéral eau/solvant organique est généralement compris entre 2/1 et 20/1 de préférence compris entre 2/1 et 5/1. Le mélange obtenu à l'issue de l'étape a) peut comporter de 70 à 95 %, de préférence de 80 à 90 % en poids de solvant, de 4 à 20 %, de préférence de 8 à 15 % en poids de cyclodextrines et de 0,5 à 10 %, de préférence de 0,5 à 5 % en poids de peroxyde organique.

Les cyclodextrines et le peroxyde organique sont généralement introduits dans le solvant sous agitation. Ledit peroxyde organique peut alors se présenter sous forme d'une poudre ou en solution dans un des solvants cités ci-dessus.

L'étape b) de chauffage du mélange dure généralement de une à dix heures, de préférence de une à quatre heures.

La récupération du composé d'inclusion peut se faire soit par lyophilisation du mélange, soit par diminution de la température du mélange de manière à obtenir un précipité constitué desdits composés d'inclusion.

Dans ce dernier cas, on abaisse généralement la température au plus jusqu'à 0° C, de préférence entre 5 et 10° C. Le temps d'abaissement de la température n'est pas critique mais est généralement supérieur à une heure, de préférence compris entre trois et huit heures. Puis on sépare ledit précipité du mélange, par des méthodes classiques, par exemple par filtration.

Le précipité séparé peut être alors séché pour obtenir une poudre.

Un deuxième procédé de préparation des composés d'inclusion selon l'invention consiste en les étapes suivantes :

a) On mélange dans un solvant des cyclodextrines et un peroxyde organique de sorte à obtenir une pâte,

b) on sèche la pâte jusqu'à obtention d'un produit solide,

c) On broie ledit produit solide jusqu'à obtention d'une poudre.

La pâte obtenue à l'étape a) comporte de 20 à 60 %, de préférence de 40 à 50 % en poids de solvant, de 30 à 70 %, de préférence de 40 à 55 % en poids de cyclodextrines et de 1 à 15 %, de préférence de 4 à 10 % en poids de peroxyde organique.

A titre de solvant on peut employer ceux cités dans le cadre du premier procédé décrit ci-dessus. L'eau est cependant un solvant préféré.

Le peroxyde organique peut se présenter soit sous forme de poudre, soit être en mélange avec au moins une partie dudit solvant.

On mélange généralement le solvant, les cyclodextrines et le peroxyde organique pendant au moins trente minutes, de préférence pendant trois à dix heures et plus préférentiellement pendant cinq à huit heures. Le mélange peut se faire à température ambiante.

Les composés d'inclusion selon la présente invention peuvent être utilisés en tant que médicament et notamment pour la préparation d'un médicament destiné à soigner l'acnée.

Ils peuvent également être utilisés dans l'industrie alimentaire, notamment comme agent de blanchiment en particulier des farines, des huiles, des cires ou de lait destiné à la préparation de certains fromages. Les composés d'inclusion selon l'invention peuvent aussi être utilisés dans des compositions cosmétiques, notamment des compositions cosmétiques destinées au nettoyage de la peau.

Pour les utilisations et applications mentionnés ci-dessus on emploie de préférence des composés d'inclusion comportant du peroxyde de dibenzoyle.

Les composés d'inclusions selon l'invention, en particulier ceux enfermant du peroxyde de benzoyle et du peroxyde de dioctanoyle peuvent être aussi employés en tant qu'initiateur de polymérisation ou de copolymérisation de monomères vinyliques ou de diènes, tels l'éthylène, le chlorure de vinyle, le styrène, l'acide acrylique et ses esters, l'acide méthacrylique et ses esters, l'acétate de vinyle, l'acrylonitrile et le butadiène. Lesdits composés d'inclusion sont plus particulièrement avantageux lorsqu'ils sont employés pour la polymérisation et la copolymérisation en phase aqueuse de monomères vinyliques hydrosolubles.

Les exemples suivants ont pour but d'illustrer la présente invention :

## EXEMPE 1

On introduit dans un malaxeur Guittard ® de type ML2, 120 g d'eau, puis 100 g de cyclodextrines β commercialisées par la société Rhône-Poulenc sous la marque Rhodocap-N®. On ajoute ensuite à la solution 10 g de peroxyde de dibenzoyle. On mélange pendant sept heures, puis on sèche la pâte obtenue en l'abandonnant à température ambiante jusqu'à obtention d'un produit solide.

Ce dernier est broyé pour donner une poudre constituée des composés d'inclusion de β cyclodextrines enfermant du peroxyde de dibenzoyle.

## EXEMPLES 2 A 8

On introduit dans un solvant qui peut être un mélange eau/éthanol ou un mélange eau/acétone, des cyclodextrines β (βCD) de même nature que celles de l'exemple 1. A cette solution on ajoute alors soit du peroxyde de dibenzoyle (PDB), soit du peroxyde de dioctanoyle (PDO), en poudre. On agite le mélange ainsi obtenu pendant deux heures à des températures variables. A la fin de la période d'agitation on diminue la température d'une manière constante jusqu'à 5° C pendant cinq heures. On obtient alors un précipité des composés d'inclusion que l'on isole du mélange par filtration. Le précipité récupéré est séché dans une étuve à 40° C pendant deux heures.

On dose alors le taux de peroxyde enfermé dans le composé d'inclusion de cyclodextrine β selon la méthode décrite ci-dessous.

Les paramètres réactionnels ainsi que le taux de peroxyde enfermé dans le composé d'inclusion exprimé en % en poids par rapport au poids total dudit précipité séché (titre) figurent dans le tableau I ci-dessous.

## TABLEAU I

| EXEMPLE | EAU (en g) | ETHANOL (en g) | ACETONE (en g) | β C D (en g) | NATURE DU PEROXYDE | POIDS EN PEROXYDE (en g) | TEMPERATURE D'AGITATION (° C) | TITRE (%) |
|---|---|---|---|---|---|---|---|---|
| 2 | 80 | 20 | - | 10 | PDB | 1 | 40 | 13,6 |
| 3 | 70 | 30 | - | 10 | PDB | 1 | 40 | 15,9 |
| 4 | 80 | - | 20 | 10 | PDB | 1 | 40 | 16,3 |
| 5 | 80 | - | 20 | 10 | PDB | 2 | 40 | 18,6 |
| 6 | 280 | 120 | - | 40 | PDB | 4 | 60 | 12,8 |
| 7 | 280 | 120 | - | 40 | PDB | 6 | 40 | 14,5 |
| 8 | 280 | 120 | - | 40 | PDO | 4 | 40 | 11,0 |

Méthode de dosage du titre en peroxyde enfermé dans les composés d'inclusion :

Dans une fiole conique de 200 ml contenant 20 ml d'acétone, on introduit sous agitation 90 mg du composé d'inclusion enfermant le peroxyde à doser. On ajoute 3 ml d'une solution aqueuse à 50 % d'iodure de potassium. Une couleur rose se développe. On titre l'iode libéré au moyen d'une solution de thiosulfate de sodium 0,1 N, jusqu'à disparition de la couleur rose.

Le titre en peroxyde est alors déterminé par la formule suivante :

titre = a.n.M.0,555, dans laquelle :

– a est le volume de la solution de thiosulfate de sodium utilisée

– n est la normalité de la solution de thiosulfate de sodium

– M est le poids moléculaire du peroxyde dosé

EXEMPLE 9

On dissout dans l'eau le composé d'inclusion de β cyclodextrine enfermant du peroxyde de dibenzoyle tel qu'obtenu dans l'exemple 7. On détermine par absorption U.V. que le seuil de solubilité dans l'eau dudit composé d'inclusion est de 44 mg/l ce qui correspond à une teneur de 6 mg en peroxyde. A titre comparatif on a trouvé que le seuil de solubilité dans l'eau d'une poudre du même peroxyde, est inférieur à 3 mg/l. Les composés d'inclusion selon l'invention permettent donc d'augmenter la solubilité des peroxydes dans l'eau d'une manière importante.

## Revendications

**1** - Composés d'inclusion de cyclodextrines caractérisés en ce qu'ils consistent en des cyclodextrines enfermant des peroxydes organiques de formule $R_1$-O-O-$R_2$ dans laquelle $R_1$ et $R_2$ sont identiques ou différents et représentent $R_3$ ou

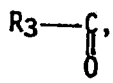

$R_3$ étant un reste hydrocarboné.

**2** - Composés d'inclusion selon la revendication 1 caractérisés en ce que lesdites cyclodextrines sont des cyclodextrines α, β, γ ou δ, des cyclodextrines α, β, γ ou δ modifiées par des groupes acyles, alkyles, hydroxyalkyles, glucosyles ou maltosyles, des mélanges desdites cyclodextrines, ou des mélanges desdites cyclodextrines et de dextrines ou d'oligosaccharides linéaires.

**3** - Composés d'inclusion selon l'une des revendications 1 et 2 caractérisés en ce que lesdites cyclodextrines sont des cyclodextrines β.

**4** - Composés d'inclusion selon l'une des revendications 1 à 3 caractérisés en ce que $R_3$ est un radical aliphatique linéaire ou ramifié, saturé ou insaturé, ou un radical aryle.

**5** - Composés d'inclusion selon la revendication 4 caractérisé en ce que $R_3$ est un radical alkyle en $C_1$-$C_{10}$ ou un radical phényle.

**6** - Composés d'inclusion selon l'une des revendications 1 à 5 caractérisés en ce que $R_1$ et $R_2$ représentent un reste

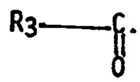

**7** - Composés d'inclusion selon l'une des revendications 1 à 6 caractérisés en ce que lesdits peroxydes organiques sont le peroxyde de dibenzoyle ou le peroxyde de dioctanoyle.

**8** - Composés d'inclusion selon l'une des revendications 1 à 7 caractérisés en ce que lesdits composés d'inclusion se présentent sous forme solide.

**9** - Procédé de préparation des composés d'inclusion selon l'une des revendication 1 à 8 caractérisés en ce qu'on effectue les étapes suivantes :

a) On solubilise les cyclodextrines dans un solvant dans lequel on ajoute le peroxyde organique antérieurement, simultanément ou postérieurement auxdites cyclodextrines.

b) On porte le mélange ainsi obtenu à une température comprise entre 25 et 70° C, de préférence comprise entre 40 et 65° C.

c) On récupère le composé d'inclusion ainsi formé.

**10** - Procédé selon la revendication 9 caractérisé en ce qu'on récupère ledit composé d'inclusion par diminution de la température dudit mélange de manière à former un précipité constitué desdits composés d'inclusion, que l'on sépare dudit mélange.

**11** - Procédé selon la revendication 9 caractérisé en ce qu'on récupère ledit composé d'inclusion par lyophilisation dudit mélange.

**12** - Procédé selon l'une des revendications 9 à 11 caractérisés en ce que ledit solvant est l'eau ou un mélange d'eau et d'au moins un solvant organique miscible à l'eau.

**13** - Procédé selon la revendication 12 caractérisé en ce que ledit solvant organique est un alcool comme le méthanol, l'éthanol, le n-propanol ou l'isopropanol, une cétone comme l'acétone ou un éther comme le dioxanne.

**14** - Procédé selon l'une des revendications 9 à 13 caractérisés en ce que le mélange obtenu à l'étape a) comporte de 70 à 95 %, de préférence de 80 à 90 % en poids de solvant, de 4 à 20 %, de préférence de 8 à 15 % en poids de cyclodextrines et de 0,5 à 10 %, de préférence de 0,5 à 5 % en poids de peroxyde organique.

**15** - Procédé de préparation des composés d'inclusion selon l'une des revendications 1 à 8 caractérisés en ce qu'on effectue les étapes suivantes :

a) On mélange dans un solvant des cyclodextrines et un peroxyde organique de sorte à obtenir un pâte,

b) On sèche la pâte jusqu'à obtention d'un produit solide,

c) On broie ledit produit solide jusqu'à obtention d'une poudre constituée par lesdits composés d'inclusion.

**16** - Procédé selon la revendication 15 caractérisé en ce que ledit solvant est l'eau.

**17** - Procédé selon l'une des revendications 15 et 16 caractérisés en ce que la pâte obtenue à l'étape a) comporte de 20 à 60 %, de préférence de 40 à 50 % en poids de solvant, de 30 à 70 %, de préférence de 40 à 55 % en poids de cyclodextrines et de 1 à 15 %, de préférence de 4 à 10 % en poids de peroxyde organique.

**18** - Utilisation en tant que médicaments des composés d'inclusion selon l'une des revendications 1 à 8 ou des composés d'inclusion tels qu'obtenus selon les procédés des revendications 9 à 17.

**19** - Utilisation des composés d'inclusion selon l'une des revendications 1 à 8 ou des composés d'inclusion tels qu'obtenus selon les procédés des revendications 9 à 17 pour la préparation d'un médicament destiner à soigner l'acnée.

**20** - Utilisation des composés d'inclusion selon l'une des revendications 1 à 8 ou des composés d'inclusion tels qu'obtenus selon les procédés des revendications 9 à 17 dans l'industrie alimentaire, notamment en tant qu'agent de blanchiment.

**21** - Utilisation des composés d'inclusion selon l'une des revendications 1 à 8 ou des composés d'inclusion tels qu'obtenues selon les procédés des revendications 9 à 17 dans des composition cosmétiques, notamment des compositions cosmétiques destinées au nettoyage de la peau.

**22** - Utilisations selon l'une des revendications 18 à 21 des composés d'inclusion selon l'une des revendications 1 à 8 ou des composés d'inclusion tels qu'obtenus selon le procédé des revendications 9 à 17 caractérisés en ce que lesdits composés d'inclusion enferment du peroxyde de dibenzoyle.

**23** - Utilisation des composés d'inclusion selon l'une des revendications 1 à 8 ou des composés d'inclusion selon l'une des revendications 9 à 17 en tant qu'initiateur de polymérisation ou de copolymérisation de monomères vinyliques ou de diènes.

**24** - Utilisation selon la revendication 23 pour la polymérisation ou la copolymérisation de monomères vinyliques hydrosolubles, en phase aqueuse.